# EUROPEAN PATENT APPLICATION

(11) **EP 4 535 004 A1**
(43) Date of publication of application: **09.04.2025**
(21) Application number: 23811896.2
(22) Date of filing: 26.05.2023
(51) Int. Cl.: G01N 33/68

(54) **METHOD FOR ASSISTING DIAGNOSIS OF BLOOD TUMOR, METHOD FOR OBTAINING DATA FOR DIAGNOSING BLOOD TUMOR, AND KIT FOR SAID METHODS**

(30) Priority: 27.05.2022 JP 2022087037
(71) Applicant: FUJIFILM Corporation, Tokyo 106-8620 (JP); Fujita Academy, Toyoake-shi, Aichi 470-1192 (JP)
(72) Inventor: SAITO Kuniaki, Toyoake-shi, Aichi 470-1192 (JP); YAMAMOTO Yasuko, Toyoake-shi, Aichi 470-1192 (JP); KAMBARA Kengo, Tokyo 106-0031 (JP)
(74) Representative: Dehns Germany Partnerschaft mbB
(86) International application number: PCT/JP2023/019625
(87) International publication number: WO 2023/229024

(57) **Abstract**

There are provided a method of assisting diagnosis of a hematological tumor, including using, as an indicator, a decrease in an amount of kynurenic acid in a subject-derived specimen, or using, as an indicator, a ratio between at least one amount selected from the group consisting of an amount of anthranilic acid, an amount of kynurenine, an amount of 3-hydroxyanthranilic acid, an amount of 3-hydroxykynurenine, and an amount of tryptophan, and the amount of kynurenic acid in the subject-derived specimen, a method of obtaining data for diagnosis of a hematological tumor, and a kit.

## Description

### Technical Field

The present invention relates to a method of assisting diagnosis of a hematological tumor, a method of obtaining data for diagnosis of a hematological tumor, and a kit for these methods

### Background Art

Hematological tumors such as a lymphatic hematological tumor and a myeloid hematological tumor are difficult to treat with a surgical operation generally carried out for, for example, tumors of other organs, and thus a medical treatment with a drug such as an anti-cancer agent, a radiation therapy using X-rays, γ-rays, or the like, a medical treatment with bone marrow transplantation, and the like are carried out.

All of these medical treatments are very burdensome for patients. Therefore, an accurate diagnosis should be made before the medical treatment. Therefore, there is a demand for the development of a method of assisting diagnosis of a hematological tumor, which has an excellent degree of certainty (sensitivity and specificity), and a method of obtaining data for diagnosis of a hematological tumor, which has an excellent degree of certainty (sensitivity and specificity)

For example, in myelodysplastic syndrome, it has been reported that the blood concentration of tryptophan (Trp) decreases and the blood concentration of kynurenine (Kyn) as a metabolic product of the tryptophan increases, which is known to be capable of being a biomarker for assisting diagnosis of myelodysplastic syndrome (Non-Patent Document 1).

### Prior Art Documents

### Non-Patent Documents

Non-Patent Document 1: C. Berthon et al., Leukemia Research 37 (2013) 573-579

### SUMMARY OF THE INVENTION

### Object to be solved by the invention

However, C. Berthon et al., Leukemia Research 37 (2013) 573-579 only describes a relationship between an increase in the Trp metabolic product and myelodysplastic syndrome, which is a specific hematological tumor, and it has not studied the application to the diagnosis of other hematological tumors.

An object of the present invention is to provide a method of assisting diagnosis of a hematological tumor, which has an excellent degree of certainty (sensitivity and specificity), and a method of obtaining data for diagnosis of a hematological tumor, which has an excellent degree of certainty (sensitivity and specificity), and a kit for these methods.

### Means for solving the object

The inventors of the present invention focused on Trp and kynurenic acid (KA) which is a metabolic product generated from Trp, as well as anthranilic acid (AA), kynurenine (Kyn), 3-hydroxyanthranilic acid (3-HAA), and 3-hydroxykynurenine (3-HKyn), in the Trp pathway and the Kyn pathway which is a metabolic pathway of Trp. Then, as a result of evaluating the performance of these substances as a biomarker for malignant lymphoma, it has been found that these substances serve as excellent diagnostic markers for a hematological tumor, whereby the present invention has been completed.

An example of the Kyn pathway is shown below. However, the following figure is merely an example of the Kyn pathway, and there is a possibility that other enzymes are involved and there is a possibility that other intermediates are present. In addition, in the Kyn pathway, each metabolic product may be further metabolized.

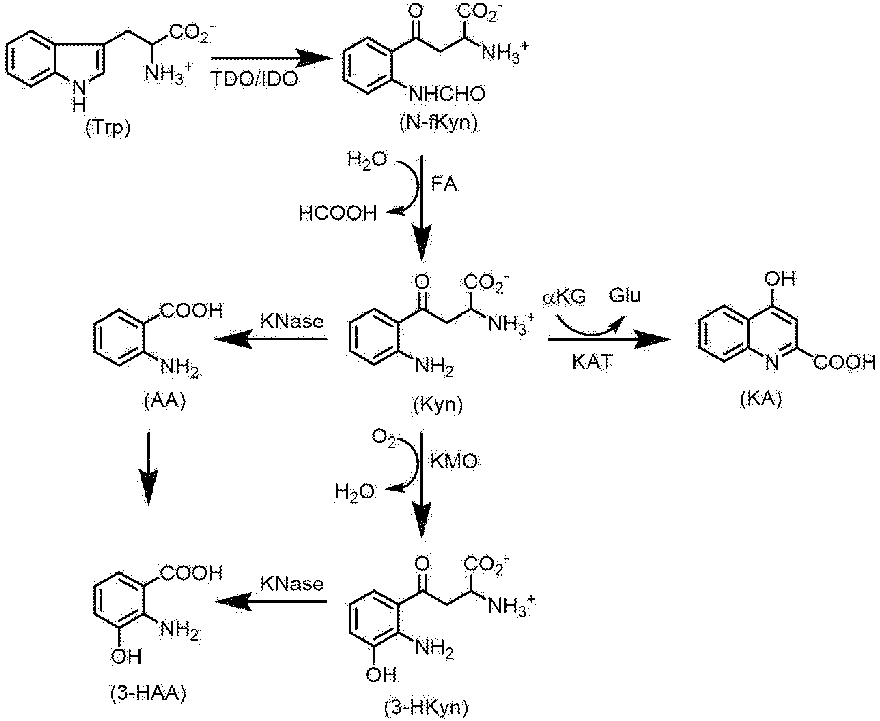

Details of the abbreviations in the above formula are as follows.
Trp: tryptophan
TDO: tryptophan 2,3-dioxygenase
IDO: indoleamine 2,3-dioxygenase
N-fKyn: N-formylkynurenine
FA: formamidase
Kyn: kynurenine
KAT: kynurenine aminotransferase
αKG: α-ketoglutaric acid
Glu: glutamic acid
KA: kynurenic acid
KNase: kynureninase
AA: anthranilic acid
3-HAA: 3-hydroxyanthranilic acid
KMO: kynurenine 3-monooxygenase
3-HKyn: 3-hydroxykynurenine

Representative aspects of the present invention are as follows.
<1> A method of assisting diagnosis of a hematological tumor, comprising:
   using, as an indicator, a decrease in an amount of kynurenic acid in a subject-derived specimen; or
   using, as an indicator, a ratio between at least one amount selected from the group consisting of an amount of anthranilic acid, an amount of kynurenine, an amount of 3-hydroxyanthranilic acid, an amount of 3-hydroxykynurenine, and an amount of tryptophan, and the amount of kynurenic acid in the subject-derived specimen.
<2> The method of assisting diagnosis of a hematological tumor according to <1>, in which the ratio is used as an indicator.
<3> The method of assisting diagnosis of a hematological tumor according to <1> or <2>, comprising:
   the following (1), (2), and (3);
   (1) measuring A, which is the amount of kynurenic acid, and B, which is at least one amount selected from the group consisting of the amount of anthranilic acid, the amount of kynurenine, the amount of 3-hydroxyanthranilic acid, the amount of 3-hydroxykynurenine, and the amount of tryptophan in the subject-derived specimen,
   (2) (i) calculating B/A, which is a ratio of B to A obtained in (1) or (ii) calculating A/B, which is a ratio of A to B obtained in (1), and
   (3) providing an indicator for diagnosing whether or not a subject has a hematological tumor based on a result of (2).
<4> The method of assisting diagnosis of a hematological tumor according to <3>, further comprising:
   comparing at least one of the A/B or the B/A with a reference value.
<5> The method of assisting diagnosis of a hematological tumor according to <3>, in which the (2) is calculating a ratio of the amount of kynurenic acid to the amount of anthranilic acid, or a ratio of the amount of anthranilic acid to the amount of kynurenic acid.
<6> The method of assisting diagnosis of a hematological tumor according to any one of <1> to <5>, in which the hematological tumor is a lymphatic hematological tumor or a myeloid hematological tumor.
<7> The method of assisting diagnosis of a hematological tumor according to any one of <1> to <6>, in which the hematological tumor is a lymphatic hematological tumor.
<8> The method of assisting diagnosis of a hematological tumor according to <6> or <7>, in which the lymphatic hematological tumor is adult T-cell leukemia, diffuse large B-cell lymphoma, extranodal NK/T-cell lymphoma, nasal type follicular lymphoma, mucosal-associated lymphoid tissue lymphoma, mantle cell lymphoma, or Hodgkin's lymphoma.
<9> The method of assisting diagnosis of a hematological tumor according to any one of <1> to <8>, in which the specimen is serum, blood plasma, or whole blood.
<10> A method of obtaining data for diagnosis of a hematological tumor, the method comprising:
   the following (1) and (2);
   (1) measuring A which is an amount of kynurenic acid or measuring A which is an amount of kynurenic acid and B which is at least one amount selected from the group consisting of an amount of anthranilic acid, an amount of kynurenine, an amount of 3-hydroxyanthranilic acid, an amount of 3-hydroxykynurenine, and an amount of tryptophan in a subject-derived specimen, and
   (2) (i) using A obtained in the (1) as data for diagnosing a hematological tumor, (ii) calculating B/A, which is a ratio of B to A obtained in the (1), and using the calculated B/A as data for diagnosing a hematological tumor, or (iii) calculating A/B, which is a ratio of A to B obtained in the (1), and using the calculated A/B as data for diagnosing a hematological tumor.
<11> A kit for carrying out the method of assisting diagnosis of a hematological tumor according to any one of <1> to <9> or the method of obtaining data for diagnosis of a hematological tumor according to <10>, the kit comprising:
   a mobile phase; and
   a column.
<12> A kit for carrying out the method of assisting diagnosis of a hematological tumor according to any one of <1> to <9> or the method of obtaining data for diagnosis of a hematological tumor according to <10>, the kit comprising:
   a substance having an affinity for kynurenic acid; or
   a substance having an affinity for kynurenic acid and
   at least one substance selected from the group consisting of a substance having an affinity for anthranilic acid, a substance having an affinity for kynurenine, a substance having an affinity for 3-hydroxyanthranilic acid, a substance having an affinity for 3-hydroxykynurenine, and a substance having an affinity for tryptophan.

### Effect of the invention

According to the present invention, there are provided a method of assisting diagnosis of a hematological tumor, which has an excellent degree of certainty (sensitivity and specificity), and a method of obtaining data for diagnosis of a hematological tumor, which has an excellent degree of certainty (sensitivity and specificity), and a kit for these methods.

### Embodiments for carrying out the invention

A method of assisting diagnosis of a hematological tumor according to an embodiment of the present invention (hereinafter, may be abbreviated as "the assisting method according to the embodiment of the present invention") includes using, as an indicator, a decrease in a KA amount in a subject-derived specimen; or using, as an indicator, a ratio of a KA amount to at least one amount selected from the group consisting of an AA amount, a Kyn amount, a 3-HAA amount, a 3-HKyn amount, and a Trp amount in the subject-derived specimen.

The inventors of the present invention have found that in a hematological tumor group, a KA amount in a specimen is decreased as compared with a healthy group, and have found that amounts of other metabolites such as a Kyn amount, a 3-HAA amount, and an AA amount are increased, thereby completing the present invention.

Hereinafter, details of the assisting method of the embodiment of the present invention will be described.

### [Hematological tumor]

The hematological tumor according to the present invention means a state in which a hematopoietic cell (for example, a hematopoietic stem cell) has become cancerous. In addition, the hematological tumor can be classified into a lymphatic hematological tumor or a myeloid hematological tumor depending on the origin of the hematopoietic cell.

The lymphatic hematological tumor is a tumor in which a cell differentiated from a lymphatic hematopoietic stem cell has become cancerous, and specific examples thereof include adult T-cell leukemia (ATLL), diffuse large B-cell lymphoma (DLBCL), extranodal NK/T-cell lymphoma (ENKL), follicular lymphoma (FL), mucosal-associated lymphoid tissue lymphoma (MALT), mantle cell lymphoma (MCL), Hodgkin's lymphoma (HL), non-Hodgkin's lymphoma, NK/T-cell lymphoma, and B-cell acute lymphoblastic leukemia (B-ALL).

On the other hand, the myeloid hematological tumor is a tumor in which a cell differentiated from a myeloid hematopoietic stem cell has become cancerous, and specific examples thereof include chronic myelomonocytic leukemia (CMMoL), myelodysplastic syndromes (MDS), chronic myelogenous leukemia (CML), and acute myelogenous leukemia (AML).

The assisting method according to the embodiment of the present invention is useful for assisting diagnosis of a lymphatic hematological tumor or a myeloid hematological tumor, and it is particularly useful for assisting diagnosis of a lymphatic hematological tumor.

In addition, among the lymphatic hematological tumors, the assisting method according to the embodiment of the present invention is preferably used for assisting diagnosis of adult T-cell leukemia, diffuse large B-cell lymphoma, extranodal NK/T-cell lymphoma, nasal type follicular lymphoma, mucosal-associated lymphoid tissue lymphoma, mantle cell lymphoma, or Hodgkin's lymphoma.

### [Subject]

The subject according to the present invention means a human who suffers from a hematological tumor or a human who is likely to have a hematological tumor.

### [Specimen]

The specimen according to the present invention may be any specimen as long as it is the above-described subject-derived specimen, and specific examples thereof include specimens derived from living bodies such as serum, blood plasma, whole blood, urine, saliva, cerebrospinal fluid, tissue fluid, sweat, tears, amniotic fluid, bone marrow fluid, pleural effusion, ascites, interstitial fluid, aqueous humor, and vitreous humor, among which a blood-derived specimen such as serum, blood plasma, or whole blood, urine, or cerebrospinal fluid is preferable, a blood-derived specimen such as serum, blood plasma, or whole blood is more preferable, and serum is particularly preferable.

A method of obtaining (collecting) a specimen according to the present invention from a subject is not particularly limited. For example, it suffices that the specimen is obtained (collected) from the subject based on a method publicly known per se, and as necessary, separation, concentration, purification, and the like may be carried out according to the method publicly known per se.

In addition, the specimen may be a specimen immediately after being collected from a subject or may be a specimen that is stored after being collected from a subject. It is noted that the method for preserving a specimen may be any method that is generally used in this field.

In addition, it is preferable that the assisting method according to the embodiment of the present invention includes using, as an indicator, a ratio of a KA amount to at least one amount selected from the group consisting of an AA amount, a Kyn amount, a 3-HAA amount, a 3-HKyn amount, and a Trp amount in the subject-derived specimen.

### · Method of measuring KA amount, AA amount, Kyn amount, 3-HAA amount, 3-HKyn amount, and Trp amount

The method of measuring the KA amount, the AA amount, the Kyn amount, the 3-HAA amount, the 3-HKyn amount, and the Trp amount in the assisting method according to the embodiment of the present invention may be any method as long as it is generally carried out in this field. Specific examples thereof include (a) a method that uses chromatography or electrophoresis, (b) a method that uses a substance having affinity, and (c) a method that uses an amino acid analyzer, where (a) a method that uses chromatography or electrophoresis or (b) a method that uses a substance having affinity is preferable, and (a) a method that uses chromatography or electrophoresis is more preferable.

The "amount" in the KA amount, the AA amount, the Kyn amount, the 3-HAA amount, the 3-HKyn amount, and the Trp amount may be any of an absolute value of a volume, a mass, or the like, or a relative value of a concentration, an ion intensity, an absorbance, a fluorescence intensity, a turbidity, or the like.

In addition, the reagent to be used for measuring the KA amount, the AA amount, the Kyn amount, the 3-HAA amount, the 3-HKyn amount, and the Trp amount may be any reagent as long as it is generally used in this field, and it suffices that the concentration and pH thereof is in a range generally used in this field. In addition, The conditions and operation methods for devices may be also any conditions and operation methods as long as they are generally used in this field.

Hereinafter, each of (a) the method that uses chromatography or electrophoresis and (b) the method that uses a substance having affinity will be specifically described.

### (a) Method that uses chromatography or electrophoresis

It suffices that (a) the method that uses chromatography or electrophoresis is carried out by injecting a mobile phase (an organic solvent-based mobile phase such as acetonitrile, methanol, tetrahydrofuran, isopropanol, or ethanol, an aqueous mobile phase such as water, phosphoric acid, formic acid, or acetic acid, or the like) and a specimen, into a device equipped with a column [an octadecylsilyl (ODS) column or the like], a detector (a fluorescence detector, an electrochemical detector, an ultraviolet-visible spectrophotometer, or the like) or the like, and then separating and detecting KA, AA, Kyn, 3-HAA, 3-HKyn, or Trp.

It is noted that a specific method for the method that uses chromatography or electrophoresis needs only to be carried out according to a method publicly known per se.

Specific examples of the method that uses chromatography or electrophoresis include a method that uses high performance liquid chromatography (HPLC), a method that uses gas chromatography (GC), a method that uses a liquid chromatography mass spectrometer (LC/MS), a method that uses a capillary electrophoresis mass spectrometer (CE/MS), a method that uses a gas chromatography mass spectrometer (GC/MS), a method that uses an inductively coupled plasma mass spectrometer (ICP/MS), a method that uses a liquid chromatography tandem type mass spectrometer (LC/MS/MS), and a method that uses a gas chromatography tandem type mass spectrometer (GC/MS/MS), where a method that uses high performance liquid chromatography (HPLC) or a method that uses a liquid chromatography tandem type mass spectrometer (LC/MS/MS) is preferable.

For the detection in the method that uses high performance liquid chromatography (HPLC) or the method that uses gas chromatography (GC), any method can be used as long as it is publicly known per se. Examples of the detection include, in the case of the method that uses high performance liquid chromatography (HPLC), ultraviolet-visible absorbance detection, diode array detection, fluorescence detection, and differential refractive index detection, among which ultraviolet-visible absorbance detection or fluorescence detection is preferable. In the case of the method that uses gas chromatography (GC), examples of the detection include thermal conductivity detection (TCD), hydrogen flame ionization detection (FID), electron capture type detection (ECD), flame photometric detection (FPD), and alkali thermal ionization detection (FTD). Among these, ultraviolet-visible absorbance detection and fluorescence detection are preferable.

### (b) Method that uses substance having affinity

(b) the method that uses substance having affinity is carried out by using a substance having an affinity for each of KA, AA, Kyn, 3-HAA, and Trp. Specific examples thereof include methods according to immunological measuring methods such as an enzyme-linked immunosorbent assay (ELISA method), an enzyme immunoassay (EIA method), a radioimmunoassay (RIA method), a fluoroimmunoassay (FIA method), a chemiluminescence immunoassay (CLEIA method), immunoturbidimetry such as a latex immunoturbidimetric assay, immunonephelometry, an immunochromatographic assay, Western blotting, a luminescent oxygen channeling immunoassay (LOCI method), and a liquid-phase binding assay-electrokinetic analyte transport assay (LBA-EATA method).

Specifically, the method according to the above-described immunological measuring method needs only to be carried out, for example, by bringing a substance having an affinity for KA into contact with KA to form a complex between the substance having an affinity for KA and KA, and measuring the amount of the complex. The AA amount, the Kyn amount, the 3-HAA amount, the 3-HKyn amount, and the Trp amount can also be measured according to the above-described method.

Specific examples of the substance having an affinity for KA, the substance having an affinity for AA, the substance having an affinity for Kyn, the substance having an affinity for 3-HAA, the substance having an affinity for 3-HKyn, and the substance having an affinity for Trp include an antibody, a lectin, and a nucleic acid, where an anti-KA antibody, an anti-AA antibody, an anti-Kyn antibody, an anti-3-HAA antibody, an anti-3-HKyn antibody, and an anti-Trp antibody are respectively preferable.

The above-described antibody may be any of a polyclonal antibody or a monoclonal antibody, where these may be used alone or in a combination thereof.

The above-described antibody may be an antibody fragment such as Fab, F(ab')₂, Fv, or sFv, a synthetic antibody such as a diabody, a triabody, or tetrabody, or the like.

In addition, the above-described used may be a commercially available antibody or one prepared according to a method publicly known per se.

It is noted that in a case of preparing the anti-KA antibody, the anti-AA antibody, the anti-Kyn antibody, the anti-3-HAA antibody, the anti-3-HKyn antibody, and the anti-Trp antibody according to the method publicly known per se, the preparation thereof needs only to be carried out according to, for example, "Immunoassay" (edited by the Biochemical Assay Society of JAPAN, Kodansha Ltd., 2014) or the method described in JP2018-501202A.

Further, the anti-KA antibody, the anti-AA antibody, the anti-Kyn antibody, the anti-3-HAA antibody, the anti-3-HKyn antibody, and the anti-Trp antibody may be labeled with a labeling substance.

Specific examples of the labeling substance include enzymes such as horseradish peroxidase (HRP), calf intestinal alkaline phosphatase, and β-galactosidase, radioactive isotopes such as ^{99m}Tc, ¹³¹I, ¹²⁵I, ¹⁴C, ³H, ³²P, and ³⁵S, fluorescent substances such as fluorescein, fluorescein isothiocyanate (FITC), 4-methylumbelliferone, rhodamine, or derivatives thereof, luminescent substances such as luciferin, luminol, and ruthenium complex, substances having absorption in the ultraviolet region, such as phenol, naphthol, anthracene, or a derivative thereof, substances having properties as spin labeling agents typified by a compound having an oxyl group such as 4-amino-2,2,6,6-tetramethylpiperidine-1-oxyl, dyes such as a HiLyte type dye, an Alexa type dye, and a CyDye type dye, and nanoparticles such as colloidal gold and quantum dots.

It is noted that the method of binding the labeling substance to the anti-KA antibody, the anti-AA antibody, the anti-Kyn antibody, the anti-3-HAA antibody, the anti-3-HKyn antibody, and the anti-Trp antibody needs only to be carried out according to a method publicly known per se.

The method of measuring the amount of the complex between the substance having an affinity for KA and KA, the amount of the complex between the substance having an affinity for AA and AA, the amount of the complex between the substance having an affinity for Kyn and Kyn, the amount of the complex between the substance having an affinity for 3-HAA and 3-HAA, the amount of the complex between the substance having an affinity for 3-HKyn and 3-HKyn, and the amount of the complex between the substance having an affinity for Trp and Trp may be any method as long as it is a method that can measure the amounts of the complexes described above, and specifically, the method needs only to be carried out by detecting each of the signals derived from labeling substances bound to KA or the substance having an affinity for KA, AA or the substance having an affinity for AA, Kyn or the substance having an affinity for Kyn, 3-HAA or the substance having an affinity for 3-HAA, 3-HKyn or the substance having an affinity for 3-HKyn, and Trp or the substance having an affinity for Trp.

Specifically, the method of detecting each of the signals derived from the above-described labeling substances needs only to be carried out, for example, by detecting the signal derived from the above-described labeling substance by a method publicly known per se. For example, in a case where the labeling substance is an enzyme, the measurement needs only to be carried out according to a conventional method of immunoassay, for example, a method disclosed in "Enzyme Immunoassay" (Protein, Nucleic Acid and Enzyme, Separate Volume No. 31, edited by Tsunehiro Kitagawa, Toshio Nanbara, Akio Tsuji, and Eiji Ishikawa, pp. 51-63, Kyoritsu Shuppan Co., Ltd., 1987), and in a case where the labeling substance is a radioactive substance, the measurement may be carried out, for example, according to a conventional method which is carried out by a radioimmunoassay (RIA), and by appropriately selecting and using a measurement apparatus such as an immersion GM counter, a liquid scintillation counter, a well-type scintillation counter, or a counter for HPLC, depending on the type and intensity of radiation generated by the radioactive substance (see, for example, "Course on Experimental Medical Chemistry", Vol. 8, supervised by Yuichi Yamamura, the 1st edition, Nakayama Shoten Co., Ltd., 1971). In addition, in a case where the labeling substance is a fluorescent substance, the measurement needs only to be carried out according to a conventional method which is carried out by a Fluorescence immunoassay (FIA) using a measurement apparatus such as a fluorophotometer, for example, according to a method disclosed in "Illustrative Description of Fluorescent Antibody", Akira Kawaoi, the 1st edition, Soft Science Inc., 1983; and in a case where the labeling substance is a luminescent substance, the measurement may be carried out according to a conventional method using a measurement apparatus such as a photo counter, for example, according to a method disclosed in "Enzyme Immunoassay" (Protein, Nucleic Acid, and Enzyme, Separate Volume No. 31, edited by Tsunehiro Kitagawa, Toshio Nanbara, Akio Tsuji, and Eiji Ishikawa, pp. 252-263, Kyoritsu Shuppan Co., Ltd., 1987). Further, in a case where the labeling substance is a substance which has an absorption in the ultraviolet region, the measurement needs only to be carried out by a conventional method using a measurement apparatus such as a spectrophotometer; and in a case where the labeling substance has a property of spin, the measurement may be carried out by a conventional method using electron spin resonance equipment, for example, according to a method disclosed in "Enzyme Immunoassay" (Protein, Nucleic Acid, and Enzyme, Separate Volume No. 31, Tsunehiro Kitagawa, Toshio Nanbara, Akio Tsuji, and Eiji Ishikawa, pp. 264-271, Kyoritsu Shuppan Co., Ltd., 1987).
· In regard to a ratio of a KA amount to at least one amount selected from the group consisting of an AA amount, a Kyn amount, a 3-HAA amount, a 3-HKyn amount, and a Trp amount in the subject-derived specimen.

In the assisting method according to the embodiment of the present invention, a ratio of a KA amount to at least one amount selected from the group consisting of an AA amount, a Kyn amount, a 3-HAA amount, a 3-HKyn amount, and a Trp amount in the subject-derived specimen includes
(i) B/A, which is a ratio of B which is at least one amount selected from the group consisting of an AA amount, a Kyn amount, a 3-HAA amount, a 3-HKyn amount, and a Trp amount, to A which is a Kyn amount, or
(ii) A/B, which is a ratio of A which is a KA amount to B which is at least one amount selected from the group consisting of an AA amount, a Kyn amount, a 3-HAA amount, a 3-HKyn amount, and a Trp amount.

In the above-described method, specific examples of the method of measuring the KA amount, the AA amount, the Kyn amount, the 3-HAA amount, the 3-HKyn amount, or the Trp amount are as described in the section of "Method of measuring KA amount, AA amount, Kyn amount, 3-HAA amount, 3-HKyn amount, and Trp amount" described above.

Examples of the method of determining "the ratio of the KA amount to at least one amount selected from the group consisting of the AA amount, the Kyn amount, the 3-HAA amount, the 3-HKyn amount, and the Trp amount in the subject-derived specimen" described above include the following method.

The method includes
(i) calculating B/A, which is a ratio of B which is at least one amount selected from the group consisting of an AA amount, a Kyn amount, a 3-HAA amount, a 3-HKyn amount, and a Trp amount, to A which is a Kyn amount, or
(ii) calculating A/B, which is a ratio of A which is a KA amount to B which is at least one amount selected from the group consisting of an AA amount, a Kyn amount, a 3-HAA amount, a 3-HKyn amount, and a Trp amount.

Specific examples thereof include the following method.
(i) dividing the AA amount, the Kyn amount, the 3-HAA amount, the 3-HKyn amount, or the Trp amount by the KA amount to calculate a ratio (AA/KA) of the AA amount to the KA amount, or
(ii) dividing KA by the AA amount, the Kyn amount, the 3-HAA amount, the 3-HKyn amount, or the Trp amount to calculate a ratio (KA/AA) of the KA amount to the AA amount, a ratio (KA/Kyn) of the KA amount to the Kyn amount, a ratio (KA/3-HAA) of the KA amount to the 3-HAA amount, a ratio (KA/3-HKyn) of the KA amount to the 3-HKyn amount, or a ratio (KA/Trp) of the KA amount to the Trp amount.

Among these, "The ratio of the KA amount to at least one amount selected from the group consisting of the AA amount, the Kyn amount, the 3-HAA amount, the 3-HKyn amount, and the Trp amount in the subject-derived specimen" described above is preferably AA/KA, KA/AA, KA/Kyn, KA/3-HAA, and KA/Trp, more preferably AA/KA, KA/AA, KA/Kyn, KA/3-HAA, or KA/Trp, still more preferably AA/KA, KA/AA, KA/Kyn, or KA/3-HAA, and particularly preferably AA/KA and KA/AA.

It is preferable that the assisting method according to the embodiment of the present invention includes the following (1), (2), and (3).
(1) measuring A, which is the amount of kynurenic acid, and B, which is at least one amount selected from the group consisting of the amount of anthranilic acid, the amount of kynurenine, the amount of 3-hydroxyanthranilic acid, the amount of 3-hydroxykynurenine, and the amount of tryptophan in the subject-derived specimen,
(2) (i) calculating B/A, which is a ratio of B to A obtained in the (1) or (ii) calculating A/B, which is a ratio of A to B obtained in the (1), and
(3) providing an indicator for diagnosing whether or not a subject has a hematological tumor based on a result of the (2).

In the above-described method, specific examples of the method of measuring the KA amount, the AA amount, the Kyn amount, the 3-HAA amount, the 3-Hkyn amount, or the Trp amount, pertaining to (1), are as described in the section of "Method of measuring KA amount, AA amount, Kyn amount, 3-HAA amount, 3-Hkyn amount, and Trp amount" described above.

In addition, in the above-described method, the details of the method of calculating B/A or A/B pertaining to (2) and preferred specific examples thereof are also as described in the section of "In regard to a ratio of a KA amount to at least one amount selected from the group consisting of an AA amount, a Kyn amount, a 3-HAA amount, a 3-HKyn amount, and a Trp amount in the subject-derived specimen" described above.

(3) according to the assisting method according to the embodiment of the present invention described above makes it possible to provide an indicator for diagnosing whether or not a subject has a hematological tumor based on a result of (2) according to the present invention. The above-described indicator is preferably the amount of kynurenic acid, B/A, or A/B. In addition, it may be an indicator that is obtained by processing these values, for example, comparing a plurality of values among these values.

It is preferable that the assisting method according to the embodiment of the present invention further includes comparing the indicator provided in (3) (hereinafter, may be abbreviated as "the value derived from the subject") with a preset reference value (cut off value).

Specifically, it is preferable that the assisting method according to the embodiment of the present invention further includes comparing at least one of the above-described (A/B) or the above-described (B/A) with a reference value (cut off value).

That is, in a case of carrying out a determination based on, for example, the value of AA/KA,
(i) in a case where the value of AA/KA derived from the subject is equal to or larger than the preset reference value (cut off value or the like), it is possible to make such a determination that, for example, "the subject is likely to have a hematological tumor" or "the subject is highly likely to have a hematological tumor", or
(ii) in a case where the value derived from the subject is smaller than the preset reference value (cut off value or the like), it is possible to make such a determination that, for example, "the subject is unlikely to have a hematological tumor" or "the subject is likely to have a hematological tumor".

In addition, in a case of carrying out a determination based on, for example, the value of KA/AA,
(i) in a case where the value derived from the subject is equal to or less than the preset reference value (cut off value or the like), it is possible to make such a determination that, for example, "the subject is likely to have a hematological tumor" or "the subject is highly likely to have a hematological tumor", or
(ii) in a case where the value derived from the subject is larger than the preset reference value (cut off value or the like), it is possible to make such a determination that, for example, "the subject is unlikely to have a hematological tumor" or "the subject is likely to have a hematological tumor".

Here, the reference value in a case of carrying out a determination based on the value of AA (nmol/L)/KA (nmol/L) is preferably 0.20 to 1.00 and more preferably 0.30 to 0.90.

In addition, the reference value in a case of carrying out a determination based on the value of KA (nmol/L)/AA (nmol/L) is preferably 3.0 to 5.0 and more preferably 3.20 to 4.00.

However, the reference value may vary depending on the race, the residential area, the lifestyle, and the like of the subject.

In addition, in a case of carrying out a determination based on, for example, the value of KA, KA/Trp, KA/Kyn, or KA/3-HAA,
(i) in a case where the value derived from the subject is equal to or less than the preset reference value (cut off value or the like), it is possible to make such a determination that, for example, "the subject is likely to have a hematological tumor" or "the subject is highly likely to have a hematological tumor", or
(ii) in a case where the value derived from the subject is larger than the preset reference value (cut off value or the like), it is possible to make such a determination that, for example, "the subject is unlikely to have a hematological tumor" or "the subject is likely to have a hematological tumor".

Here, the reference value in a case of carrying out a determination based on the value (nmol/L) of KA is preferably 30.00 to 50.00 (nmol/L) and more preferably 31.00 to 43.00 (nmol/L).

The reference value in a case of carrying out a determination based on the value of KA (nmol/L)/Trp (µmol/L) is preferably 0.75 to 0.85 and more preferably 0.76 to 0.81.

The reference value in a case of carrying out a determination based on the value of the value of KA (nmol/L)/Kyn (µmol/L) is preferably 15.00 to 28.00 and more preferably 16.00 to 25.00.

The reference value in a case of carrying out a determination based on the value of KA (nmol/L)/3 - HAA (nmol/L) is preferably 1.90 to 3.50 and more preferably 2.00 to 3.00.

However, these reference values may vary depending on the race, the residential area, the lifestyle, and the like of the subject.

It is noted that the reference value (cutoff value or the like) can be determined based on a statistical analysis such as a receiver operating characteristic (ROC) curve analysis, by using the value derived from the subject suffering from a hematological tumor and "an indicator obtained from (3) according to the present invention by using an amount of kynurenic acid of a specimen derived from a healthy subject or a specimen derived from a healthy subject" (hereinafter, may be abbreviated as the value derived from the healthy subject).

In addition, the sensitivity or specificity of the reference value (the cutoff value or the like) is, for example, 60% or more, preferably 70% or more, more preferably 80% or more, and still more preferably 90% or more.

In another form, in a case of carrying out a determination based on the value of AA/KA, the value derived from the subject and the value derived from the healthy subject are compared with each other, and,
(i) in a case where the value derived from the subject is higher than the value derived from the healthy subject, it is possible to make such a determination that, for example, "the subject is likely to have a hematological tumor" or "the subject is highly likely to have a hematological tumor", or
(ii) in a case where there is no significant difference between the value derived from the subject and the value derived from the healthy subject, it is possible to make such a determination that, for example, "the subject is unlikely to have a hematological tumor" or "the subject is likely to have a hematological tumor".

In still another form, in a case of carrying out a determination based on, for example, the value of KA/AA, the value derived from the subject and the value derived from the healthy subject are compared with each other, and,
(i) in a case where the value derived from the subject is lower than the value derived from the healthy subject, it is possible to make such a determination that, for example, "the subject is likely to have a hematological tumor" or "the subject is highly likely to have a hematological tumor", or
(ii) in a case where there is no significant difference between the value derived from the subject and the value derived from the healthy subject, it is possible to make such a determination that, for example, "the subject is unlikely to have a hematological tumor" or "the subject is likely to have a hematological tumor".

In addition, in a case of carrying out a determination based on, for example, the value of KA, KA/Trp, KA/Kyn, or KA/3-HAA, the value derived from the subject and the value derived from the healthy subject are compared with each other, and
(i) in a case where the value derived from the subject is lower than the value derived from the healthy subject, it is possible to make such a determination that, for example, "the subject is likely to have a hematological tumor" or "the subject is highly likely to have a hematological tumor", or
(ii) in a case where there is no significant difference between the value derived from the subject and the value derived from the healthy subject, it is possible to make such a determination that, for example, "the subject is unlikely to have a hematological tumor" or "the subject is likely to have a hematological tumor".

In still another form, in a case of carrying out determination based on, for example, the value of AA/KA, it is possible to carry out the diagnosis of the degree of progression and the grade of malignancy of the hematological tumor, the diagnosis of the prognosis after surgery, and the like, by comparing a value derived from the subject at a certain point in time with a value derived from the subject at a different point in time in the same subject and evaluating the presence or absence of the value and/or the degree of increase or decrease of the value. That is, (i) in a case where an increase in the value is observed, it is possible to make such a determination that the pathological condition has progressed to the hematological tumor (or the grade of malignancy of the hematological tumor has increased), or a sign of the progression of the pathological condition to the hematological tumor is observed (or such a sign that the grade of malignancy of the hematological tumor has increased is observed), or (ii) it is possible to make such a determination that in a case where a decrease in the value is observed, the pathological condition of the hematological tumor has ameliorated, or a sign of the amelioration of the pathological condition of the hematological tumor is observed.

In addition, in still another form, in a case of carrying out determination based on, for example, the value of KA/AA, it is possible to carry out the diagnosis of the degree of progression and the grade of malignancy of the hematological tumor, the diagnosis of the prognosis after surgery, and the like, by comparing a value derived from the subject at a certain point in time with a value derived from the subject at a different point in time in the same subject and evaluating the presence or absence of the value and/or the degree of increase or decrease of the value. That is, (i) in a case where a decrease in the value is observed, it is possible to make such a determination that the pathological condition has progressed to the hematological tumor (or the grade of malignancy of the hematological tumor has increased), or a sign of the progression of the pathological condition to the hematological tumor is observed (or such a sign that the grade of malignancy of the hematological tumor has increased is observed), or (ii) it is possible to make such a determination that in a case where a decrease in the value is observed, the pathological condition of the hematological tumor has ameliorated, or a sign of the amelioration of the pathological condition of the hematological tumor is observed.

In addition, in a case of carrying out a determination based on, for example, the value of KA, KA/Trp, KA/Kyn, or KA/3-HAA, it is possible to carry out the diagnosis of the degree of progression and the grade of malignancy of the hematological tumor, the diagnosis of the prognosis after surgery, and the like, by comparing a value derived from the subject at a certain point in time with a value derived from the subject at a different point in time in the same subject and evaluating the presence or absence of the value and/or the degree of increase or decrease of the value. That is, (i) in a case where a decrease in the value is observed, it is possible to make such a determination that the pathological condition has progressed to the hematological tumor (or the grade of malignancy of the hematological tumor has increased), or a sign of the progression of the pathological condition to the hematological tumor is observed (or such a sign that the grade of malignancy of the hematological tumor has increased is observed), or (ii) it is possible to make such a determination that in a case where an increase in the value is observed, the pathological condition of the hematological tumor has ameliorated, or a sign of the amelioration of the pathological condition of the hematological tumor is observed.

### <Method of obtaining data for diagnosis of hematological tumor according to embodiment of present invention>

It is preferable that a method of obtaining data for diagnosis of a hematological tumor according to an embodiment of the present invention includes the following (1) and (2).
(1) measuring A which is the amount of kynurenic acid or measuring A which is an amount of kynurenic acid and B which is at least one amount selected from the group consisting of the amount of anthranilic acid, the amount of kynurenine, the amount of 3-hydroxyanthranilic acid, the amount of 3-hydroxykynurenine, and the amount of tryptophan in the subject-derived specimen, and
(2) (i) using A obtained in the (1) as data for diagnosing a hematological tumor, (ii) calculating B/A, which is a ratio of B to A in the (1), and using the calculated B/A as data for diagnosing a hematological tumor, or (iii) calculating A/B, which is a ratio of A to B obtained in the (1), and using the calculated A/B as data for diagnosing a hematological tumor.

The details of (1) in the method of obtaining data for diagnosis of a hematological tumor according to the embodiment of the present invention are the same as the above-described details of (1) in the method of assisting diagnosis of a hematological tumor according to the embodiment of the present invention.

The details of (2) in the method of obtaining data for diagnosis of a hematological tumor according to the embodiment of the present invention are respectively the same as the above-described details of (2) in the method of assisting diagnosis of a hematological tumor according to the embodiment of the present invention, except that "A" itself may be used as the data for diagnosing a hematological tumor.

In addition, the data obtained by the method of obtaining data for diagnosis of a hematological tumor according to the embodiment of the present invention can be used, for example, for the comparison with the above-described reference value (cut off value), the comparison with the value derived from the subject and the value derived from the healthy subject, or the comparison between data at different points in time in the same subject.

### <Kit according to embodiment of present invention>

A first aspect of a kit according to an embodiment of the present invention is a kit for carrying out the method of assisting diagnosis of a hematological tumor according to the embodiment of the present invention, or the method of obtaining data for diagnosis of a hematological tumor according to the embodiment of the present invention, where the kit is a kit containing a mobile phase and a column (hereinafter, may be abbreviated as a "first kit").

According to the first kit, it is possible to carry out the measurement of the amount of KA, AA, Kyn, 3-HAA, 3-HKyn, or Trp, for example, by HPLC, LC/MS, or LC/MS/MS, and it is possible to carry out the method of assisting diagnosis of a hematological tumor according to the embodiment of the present invention, or the method of obtaining data for diagnosis of a hematological tumor according to the embodiment of the present invention.

The mobile phase is not particularly limited as long as it is a publicly known mobile phase in the present field, and examples thereof include organic solvent-based mobile phases such as acetonitrile, methanol, tetrahydrofuran, isopropanol, and ethanol, and an aqueous mobile phases such as water, phosphoric acid, formic acid, and acetic acid.

The kit according to the embodiment of the present invention may further contain a buffer for deproteinization.

The buffer for deproteinization is not particularly limited and may be any buffer for deproteinization that is publicly known in the present field. Examples thereof include a buffer containing an acid such as perchloric acid, trichloroacetic acid, or metaphosphoric acid, and a buffer containing an organic solvent such as acetone, acetonitrile, methanol, or ethanol.

The column is not particularly limited, and examples thereof include an octadecylsilyl (ODS) column.

A kit according to a second aspect of the present invention is a kit for carrying out the method of assisting diagnosis of a hematological tumor according to the embodiment of the present invention, or the method of obtaining data for diagnosis of a hematological tumor according to the embodiment of the present invention, where the kit is a kit which contains a substance having affinity for kynurenic acid or a substance having affinity for kynurenic acid and contains at least one substance selected from the group consisting of a substance having an affinity for anthranilic acid, a substance having an affinity for kynurenine, a substance having an affinity for 3-hydroxyanthranilic acid, a substance having an affinity for 3-hydroxykynurenine, and a substance having an affinity for tryptophan (hereinafter, may be abbreviated as a "second kit").

According to the second kit, it is possible to carry out the measurement of the amount of KA, AA, Kyn, 3-HAA, 3-HKyn, or Trp, by "(b) Method that uses substance having affinity" described above, and it is possible to carry out the method of assisting diagnosis of a hematological tumor according to the embodiment of the present invention, or the method of obtaining data for diagnosis of a hematological tumor according to the embodiment of the present invention.

In addition, hereinafter, in a case of being simply described as "the kit according to the embodiment of the present invention", it refers to both the first kit and the second kit which are described above.

Specific examples of the substance having an affinity for KA, the substance having an affinity for AA, the substance having an affinity for Kyn, the substance having an affinity for 3-HAA, the substance having an affinity for 3-HKyn, and the substance having an affinity for Trp, which are contained in the second kit according to the present invention, include an antibody, a lectin, and a nucleic acid, where an anti-KA antibody, an anti-AA antibody, an anti-Kyn antibody, an anti-3-HAA antibody, an anti-3-HKyn antibody, and an anti-Trp antibody are preferable.

It is noted that the above-described antibody is as described in the section of "(b) Method that uses substance having affinity" of "Method of measuring KA amount, AA amount, Kyn amount, 3-HAA amount, 3-HKyn amount, and Trp amount" described above, and the same applies to specific examples, preferred examples, and the like thereof.

The second kit according to the present invention may contain reagents commonly used in this field, for example, a buffering agent, a washing agent, a reaction promoter, saccharides, a protein, salts, a stabilizer such as a surfactant, a preservative, a liquid for diluting specimens, immobilized solid phases such as an antigen-immobilized solid phase and an antibody-immobilized solid phase, a secondary antibody labeled with a labeling substance or an antibody fragments thereof, and a reagent and the like for detecting a labeling substance, where the reagent that does not impair the stability of a coexisting reagent or the like. In addition, it suffices that the concentration and pH thereof are in a range generally used in this field.

The immobilized solid phases such as the antigen-immobilized solid phase and the antibody-immobilized solid phase may be any immobilized solid phases as long as they are obtained by immobilizing KA, AA, Kyn, 3-HAA, 3-HKyn, or Trp, or an antibody such as an anti-KA antibody, an anti-AA antibody, an anti-Kyn antibody, an anti-3-HAA antibody, an anti-3-HKyn antibody, or an anti-Trp antibody, or an antibody fragment thereof, to a material such as magnetic particles such as magnetic silica particles, polystyrene, polycarbonate, polyvinyl toluene, polypropylene, polyethylene, polyvinyl chloride, nylon, polymethacrylate, gelatin, agarose, cellulose, polyethylene terephthalate, glass, or ceramic.

As the materials for the immobilized solid phases such as the antigen-immobilized solid phase and the antibody-immobilized solid phase, materials manufactured by method publicly known per se or commercially available materials may be used. For example, in a case where magnetic particles such as the magnetic silica particles described above are manufactured by a method publicly known per se, the magnetic particles can be manufactured by the method described in WO2012/173002A.

The secondary antibody labeled with the labeling substance or the antibody fragment thereof is an antibody or an antibody fragment thereof, which binds to each of an anti-KA antibody, an anti-AA antibody, an anti-Kyn antibody, an anti-3-HAA antibody, an anti-3-HKyn antibody, and an anti-Trp antibody.

It is noted that the labeling substance and the method of binding the labeling substance in the secondary antibody labeled with the labeling substance or the antibody fragment thereof are as described in the section of "(b) Method that uses substance having affinity" of "Method of measuring KA amount, AA amount, Kyn amount, 3-HAA amount, 3-HKyn amount, and Trp amount" described above, and the same applies to preferred examples, specific examples, and the like thereof.

In a case where an antibody such as an anti-KA antibody, an anti-AA antibody, an anti-Kyn antibody, an anti-3-HAA antibody, an anti-3-HKyn antibody, or an anti-Trp antibody is labeled with a labeling substance, the above-described reagent for detecting a labeling substance is a reagent for detecting a label in antibodies such as an anti-KA antibody, an anti-AA antibody, an anti-Kyn antibody, an anti-3-HAA antibody, an anti-3-HKyn antibody, and an anti-Trp antibody, or an antibody fragment thereof, which are labeled with the above-described labeling substance, or/and a label in the above-described labeled secondary antibody or an antibody fragment thereof, and examples of the substrate include a substrate for measuring the absorbance such as tetramethylbenzidine or orthophenylene diamine, a fluorescent substrate such as hydroxyphenyl propionic acid or hydroxyphenyl acetic acid, a luminescent substance such as luminol, and examples thereof a reagent for measuring absorbance such as 4-nitrophenyl phosphate, and a fluorescent substrate such as 4-methylumbelliferyl phosphate.

Further, the kit according to the embodiment of the present invention may contain an instruction manual or the like for carrying out the assisting method of the present invention. The "instruction manual" means a user's manual, attached document, pamphlet (leaflet), or the like for the kit according to the embodiment of the present invention in which the feature, the principle, the operation procedure, the determination procedure, and the like of the assisting method according to the embodiment of the present invention are substantially described in sentences and/or illustrated.

Specific examples of the kit according to the embodiment of the present invention include (i) a kit in which the details of the principles, the operation procedures, and the like of the above-described (1) and the above-described (2) in the assisting method according to the embodiment of the present invention are described, and (ii) a kit in which the details of the principles, the operation procedure, the determination procedure, and the like of the above-described (1), the above-described (2), the above-described (3) in the assisting method according to the embodiment of the present invention are described.

### <Device for assisting diagnosis of hematological tumor according to present invention>

A device for assisting diagnosis of a hematological tumor according to the present invention (hereinafter, also referred to as a device according to the present invention) includes at least a measurement unit (1) and a processing unit (2). It may further include a determination unit (3), an output unit (4), and an input unit (5).

The measurement unit (1) in the device according to the present invention is configured to measure the amount of KA or the amounts of KA and one substance selected from AA, Kyn, 3-HAA, 3-HKyn, and Trp in the specimen derived from the subject. Specific examples thereof include a device that is used in the method that uses chromatography or electrophoresis, and a device that is used in the method that uses a substance having affinity.

The processing unit (2) in the device according to the present invention is configured to (i) calculate B/A, which is a ratio of B which is at least one amount selected from the group consisting of an AA amount, a Kyn amount, a 3-HAA amount, a 3-HKyn amount, and a Trp amount, to A which is a Kyn amount, or (ii) calculate A/B, which is a ratio of A which is a KA amount to B which is at least one amount selected from the group consisting of an AA amount, a Kyn amount, a 3-HAA amount, a 3-HKyn amount, and a Trp amount, where the amounts of these substances are measured by the measurement unit (1).

It is noted that, as necessary, it may be configured to, based on the measured value measured by the measurement unit (1), calculate the KA amount, or the KA amount and at least one amount selected from the group consisting of an AA amount, a Kyn amount, a 3-HAA amount, a 3-HKyn amount, and a Trp amount and then calculate the above-described ratio based on the calculation results.

The determination unit (3) in the device according to the present invention is configured to determine whether or not a subject has a hematological tumor based on the calculation results obtained by the processing unit (2).

The output unit (4) in the device according to the present invention is configured to output the calculation result obtained by the processing unit (2) and/or the determination result obtained by the determination unit (3).

In addition, the input unit (5) in the device according to the present invention is configured to receive an operation of an operator and transmit a signal for operating the measurement unit (1) and/or the processing unit (2) to the measurement unit (1) and/or the processing unit (2).

It is noted that the measurement, calculation, determination, and the like in the measurement unit (1), the calculation unit (2), and the determination unit (3) are as described in <the assisting method according to the embodiment of the present invention>, and the same applies to preferred examples, specific examples, and the like thereof.

As described above, according to the device according to the present invention, the assisting method according to the embodiment of the present invention can be carried out easily, in a short time, and with high accuracy.

### <Method of medically treating hematological tumor according to present invention>

A method of medically treating a hematological tumor according to the present invention (hereinafter, also referred to as a therapeutic method according to the present invention) preferably includes the following (1-1), (1-2), and (1-3).
(1-1) measuring a KA amount in a specimen derived from a subject,
(1-2) determining whether or not the subject has a hematological tumor based on the result of the (1-1), and
(1-3) subjecting a subject determined to be likely to have a hematological tumor or be highly likely to have a hematological tumor, based on the result of the (1-2), to an appropriate medical treatment.

In addition, it is preferable that the therapeutic method according to the present invention includes the following (2-1), (2-2), and (2-3).
(2-1) measuring a KA amount and at least one amount selected from the group consisting of an AA amount, a Kyn amount, a 3-HAA amount, a 3-HKyn amount, and a Trp amount in a specimen derived from a subject.
(2-2) (i) calculating B/A, which is a ratio of B which is at least one amount selected from the group consisting of an AA amount, a Trp amount, a Kyn amount, a 3-HAA amount, and a 3-HKyn amount, to A which is the KA amount, or
(ii) calculating A/B; which is a ratio of A which is the KA amount to B which is at least one amount selected from the group consisting of the AA amount, the Trp amount, the Kyn amount, the 3-HAA amount, and the 3-HKyn amount.
(2-3) determining whether or not the subject has a hematological tumor based on the result of the (2-2), and
(2-4) subjecting a subject determined to be likely to have a hematological tumor or be highly likely to have a hematological tumor, based on the result of the (2-3), to an appropriate medical treatment.

Examples of the appropriate medical treatment in (1-3) or (2-4) of the therapeutic method according to the present invention include a supportive therapy such as a blood transfusion therapy or an iron removal therapy, a drug therapy carried out by administering an anti-cancer agent, an immunosuppressive agent, or the like, and transplantation of hematopoietic stem cells.

It is noted that the specimen, the hematological tumor, and the respective steps in the therapeutic method according to the present invention are as described in <the assisting method according to the embodiment of the present invention>, and the same applies to preferred examples, specific examples, and the like thereof.

Hereinafter, the present invention will be described more specifically based on examples; however, the present invention is not limited to the examples.

### Examples

### Example 1. Analysis of Trp and metabolic products thereof in hematological tumor group and healthy group

### (1) Specimen

### · Hematological tumor group

Among the medical examinees of the hematology department, 112 persons classified into the following classification types according to the diagnosis of the doctor were set as a hematological tumor group. That is, based on the guideline for medical consultation on hematopoietic tumors, the serum of a person was used as a specimen, where the person was diagnosed to have the following disease by a doctor.

### (Hematological tumor group)

ATLL: adult T-cell leukemia/lymphoma
DLBCL: diffuse large B-cell lymphoma
ENKL: extranodal NK/T-cell lymphoma, nasal type
FL: follicular lymphoma
MALT: mucosal-associated lymphoid tissue lymphoma
MCL: mantle cell lymphoma
HL: Hodgkin's lymphoma

### (Healthy group)

A person with a case in which there was no disease under the medical treatment from the biobank and there was no dosing of household medicines was classified as a healthy subject, and 43 subjects whose age was adjusted for the above-described hematological tumor group were classified into a healthy group. In addition, the serum of the case was used as a specimen.

### (2) Measurement of Trp and metabolic products of Trp in serum derived from hematological tumor group and healthy group

The concentrations of Trp and the metabolic products (Kyn, 3-HAA, KA, and AA) of the Trp in the serum derived from the hematological tumor group and the healthy group were measured by the following method.

### <Measurement of Trp, Kyn, 3-HAA, KA, and AA>

25 µL of 10% perchloric acid was added to 100 µL of each of the serum derived from the hematological tumor group and the healthy group, and deproteinization was carried out. Next, after centrifugation at 14,000 rpm and 4°C for 10 minutes, 50 µL of the supernatant was injected into a high performance liquid chromatograph [LC-20AD (manufactured by Shimadzu Corporation)], and then, Trp and Kyn in the supernatant were measured by an ultraviolet-visible spectrophotometer [SPD-20A (manufactured by Shimadzu Corporation)], and 3-HAA, KA, and AA in the supernatant were measured by a fluorescence detector [RF-10AXL (manufactured by Shimadzu Corporation)].

The conditions for the high performance liquid chromatography and the detector are as follows.
· High performance liquid chromatography [LC-20AD (manufactured by Shimadzu Corporation)]
   Mobile phase: Aqueous solution containing 10 mM (mmol/L) sodium acetate and 0.5% to 1.25% acetonitrile (pH 4.5)
   Flow rate: 0.9 mL/min
   Column: TSKgel ODS-100V, 3 µm, 4.6 mm (ID) × 15 cm (L) [manufactured by Tosoh Corporation]
   Column temperature: 40°C
· Detector
   Ultraviolet-visible spectrophotometer [SPD-20A (manufactured by Shimadzu Corporation)]:
      Trp → UV wavelength: 280 nm, Kyn → UV wavelength: 365 nm
   Fluorescence detector [RF-10AXL (manufactured by Shimadzu Corporation)]:
      3-HAA and AA → excitation wavelength: 320 nm, fluorescence wavelength: 420 nm, KA → excitation wavelength: 334 nm, fluorescence wavelength: 380 nm

### (3) Result

Table 1 shows the measurement results (average value and standard deviation) from the healthy group and the hematological tumor group, which were obtained in the (2), the P value obtained by determining the significant difference, and the presence or absence of the significant difference. Further, Table 2 shows the results obtained by calculating each of the ratio of the AA amount to the Trp amount (AA/Trp), the ratio of the AA amount to the Kyn amount (AA/Kyn), the ratio of the AA amount to the 3-HAA amount (AA/3-HAA), the ratio of the AA amount to the KA amount (AA/KA), the ratio of the KA amount to the AA amount (KA/AA), the ratio of the KA amount to the Trp amount (KA/Trp), the ratio of the KA amount to the Kyn amount (KA/Kyn), the ratio of the KA amount to the 3-HAA amount (KA/3-HAA), the ratio of the 3-HAA amount to the Trp amount (3-HAA/Trp), the ratio of the 3-HAA amount to the Kyn amount (3-HAA/Kyn), and the ratio of the Kyn amount to the Trp amount (Kyn/Trp), where the results have been obtained by using the measured values from the healthy group and the hematological tumor group, which were obtained in the (2).

A t-test or a Mann-Whitney test was used to determine the significant difference, and it was determined that there was a significant difference in a case where the P value was less than 0.05 (p-value < 0.05).

**[Table 1]**

| | | | | | A | B | C | D | E |
|---|---|---|---|---|---|---|---|---|---|
| | | | N | | Trp (µmol/L) | Kyn (µmol/L) | 3-HAA (nmol/L) | KA (nmol/L) | AA (nmol/L) |
| | All specimens | | 112 | Mean | 43.98 | 2.12 | 22.46 | 29.85 | 26.47 |
| | | | | SD | 11.962 | 1.017 | 19.324 | 16.257 | 26.050 |
| | | | | p-value | <0.0001*** | 0.004** | 0.001** | <0.0001*** | <0.0001*** |
| | | ATLL | 10 | Mean | 42.86 | 2.87 | 28.60 | 30.91 | 34.32 |
| | | | | SD | 11.018 | 0.994 | 20.821 | 13.151 | 24.444 |
| | | | | p-value | 0.008** | <0.0001*** | 0.000*** | 0.003** | <0.0001*** |
| | | DLBCL | 61 | Mean | 44.34 | 2.04 | 23.92 | 30.41 | 22.58 |
| | | | | SD | 12.881 | 0.984 | 21.818 | 17.276 | 16.014 |
| | | | | p-value | 0.000*** | 0.039* | 0.001** | <0.0001*** | <0.0001*** |
| | | ENKL | 2 | Mean | 39.37 | 2.56 | 36.30 | 17.74 | 26.66 |
| | | | | SD | 5.848 | 1.716 | 41.070 | 4.540 | 19.323 |
| Hematological tumor group | Non-Hodgkin's lymphoma | | | p-value | 0.032 | 0.612 | 0.978 | 0.004** | 0.113 |
| | | FL | 18 | Mean | 44.72 | 1.83 | 15.67 | 28.74 | 20.36 |
| | | | | SD | 9.214 | 0.814 | 8.310 | 14.404 | 10.085 |
| | | | | p-value | 0.003** | 0.492 | 0.335 | <0.0001*** | <0.0001*** |
| | | MALT | 6 | Mean | 48.45 | 1.82 | 14.68 | 30.90 | 20.13 |
| | | | | SD | 5.554 | 0.765 | 6.577 | 15.540 | 7.670 |
| | | | | p-value | 0.211 | 0.636 | 0.732 | 0.051 | 0.005** |
| | | MCL | 4 | Mean | 42.83 | 1.92 | 15.96 | 33.15 | 43.57 |
| | | | | SD | 10.169 | 1.016 | 14.345 | 23.139 | 45.539 |
| | | | | p-value | 0.069 | 0.445 | 0.956 | 0.183 | 0.000*** |
| | Hodgkin's lymphoma | HL | 11 | Mean | 40.66 | 2.55 | 23.96 | 27.98 | 48.13 |
| | | | | SD | 16.042 | 1.297 | 17.363 | 17.538 | 60.961 |
| | | | | p-value | 0.019* | 0.005** | 0.010* | <0.0001*** | 0.001** |
| Healthy group | All specimens | | 43 | Mean | 52.31 | 1.60 | 13.57 | 42.93 | 11.22 |
| | | | | SD | 7.214 | 0.345 | 4.572 | 11.505 | 3.303 |

**[Table 2]**

| | | | | | F | G | H | I-1 | I-2 | J |
|---|---|---|---|---|---|---|---|---|---|---|
| | | | N | | AA/Trp | AA/Kyn | AA/3-HAA | AA/KA | KA/AA | KA/Trp |
| | All specimens | | 112 | Mean | 0.79 | 12.80 | 1.72 | 0.96 | 3.06 | 0.78 |
| | | | | SD | 1.623 | 9.861 | 2.336 | 0.683 | 2.076 | 0.738 |
| | | | | p-value | <0.0001*** | <0.0001*** | 0.013* | <0.0001*** | <0.0001*** | 0.000*** |
| | | ATLL | 10 | Mean | 0.89 | 12.90 | 1.62 | 1.21 | 1.21 | 0.80 |
| | | | | SD | 0.691 | 11.319 | 1.308 | 0.941 | 0.687 | 0.456 |
| | | | | p-value | <0.0001*** | 0.043* | 0.339 | <0.0001*** | <0.0001*** | 0.202 |
| | | DLBCL | 61 | Mean | 0.61 | 11.69 | 1.34 | 0.85 | 1.71 | 0.78 |
| | | | | SD | 0.547 | 8.257 | 1.786 | 0.598 | 1.080 | 0.650 |
| | | | | p-value | <0.0001*** | <0.0001*** | 0.177 | <0.0001*** | <0.0001*** | <0.001** |
| | | ENKL | 2 | Mean | 0.65 | 10.19 | 1.20 | 1.41 | 0.82 | 0.45 |
| | | | | SD | 0.395 | 0.721 | 0.828 | 0.729 | 0.423 | 0.049 |
| Hematological tumor group | Non-Hodgkin's lymphoma | | | p-value | 0.004** | 0.051 | 0.549 | 0.002** | 0.002** | 0.004** |
| | | FL | 18 | Mean | 0.47 | 12.00 | 1.72 | 0.80 | 1.59 | 0.65 |
| | | | | SD | 0.236 | 6.875 | 1.214 | 0.401 | 0.848 | 0.280 |
| | | | | p-value | <0.0001*** | 0.000*** | 0.003** | <0.0001*** | 0.0001*** | 0.012* |
| | | MALT | 6 | Mean | 0.42 | 12.36 | 1.72 | 0.75 | 1.58 | 0.65 |
| | | | | SD | 0.155 | 6.345 | 1.209 | 0.342 | 0.720 | 0.384 |
| | | | | p-value | 0.003** | 0.031* | 0.047* | <0.0001*** | <0.0001*** | 0.142 |
| | | MCL | 4 | Mean | 1.05 | 24.88 | 7.38 | 1.24 | 1.02 | 0.80 |
| | | | | SD | 1.010 | 21.139 | 8.135 | 0.524 | 0.693 | 0.503 |
| | | | | p-value | <0.0001*** | 0.057 | 0.240 | <0.0001*** | <0.0001*** | 0.811 |
| | Hodgkin's lymphoma | HL | 11 | Mean | 2.35 | 16.45 | 1.97 | 1.46 | 1.06 | 1.13 |
| | | | | SD | 4.838 | 15.399 | 1.719 | 1.100 | 0.599 | 1.686 |
| | | | | p-value | 0.000*** | 0.010* | 0.094 | <0.0001*** | <0.0001*** | 0.134 |
| Healthy group | All specimens | | 43 | Mean | 0.22 | 7.27 | 0.91 | 0.28 | 4.19 | 0.82 |
| | | | | SD | 0.068 | 2.400 | 0.379 | 0.105 | 1.809 | 0.197 |

**[Table 3]**

| | | | | | K | L | M | N | O |
|---|---|---|---|---|---|---|---|---|---|
| | | | N | | KA/Kyn | KA/3-HAA | 3-HAA/Trp | 3-HAA/Kyn | Kyn/Trp |
| | All specimens | | 112 | Mean | 15.59 | 1.89 | 0.60 | 10.91 | 0.05 |
| | | | | SD | 9.686 | 1.681 | 0.795 | 6.626 | 0.041 |
| | | | | p-value | <0.0001*** | <0.0001*** | <0.0001*** | 0.110 | <0.0001*** |
| | | ATLL DLBCL | 10 | Mean | 11.35 | 1.87 | 0.67 | 10.54 | 0.07 |
| | | | | SD | 4.486 | 2.512 | 0.400 | 5.334 | 0.038 |
| | | | | p-value | <0.0001*** | <0.0001*** | <0.0001*** | 0.078 | <0.0001*** |
| | | | 61 | Mean | 16.49 | 1.64 | 0.63 | 11.89 | 0.05 |
| | | | | SD | 11.126 | 0.894 | 0.800 | 7.258 | 0.032 |
| | | | | p-value | <0.0001*** | <0.0001*** | <0.0001*** | 0.022* | 0.000*** |
| | | ENKL | 2 | Mean | 8.19 | 1.16 | 0.85 | 11.37 | 0.06 |
| Hematological tumor group | Non-Hodgkin's lymphoma | | | SD | 3.722 | 1.188 | 0.916 | 8.434 | 0.034 |
| | | | | p-value | 0.002** | 0.012* | 0.517 | 0.933 | 0.032* |
| | | FL | 18 | Mean | 17.10 | 2.34 | 0.35 | 9.31 | 0.04 |
| | | | | SD | 9.055 | 1.698 | 0.182 | 6.064 | 0.020 |
| | | | | p-value | <0.0001*** | 0.000*** | 0.058 | 0.748 | 0.016* |
| | | MALT | 6 | Mean | 17.33 | 2.37 | 0.30 | 9.46 | 0.04 |
| | | | | SD | 7.665 | 1.466 | 0.141 | 6.112 | 0.017 |
| | | | | p-value | 0.005** | 0.064 | 0.541 | 0.964 | 0.732 |
| | | MCL | 4 | Mean | 17.85 | 5.08 | 0.35 | 9.41 | 0.05 |
| | | | | SD | 9.805 | 5.482 | 0.312 | 8.391 | 0.024 |
| | | | | p-value | 0.063 | 0.985 | 0.727 | 0.840 | 0.107 |
| | Hodgkin's lymphoma | HL | 11 | Mean | 11.60 | 1.32 | 0.98 | 9.68 | 0.09 |
| | | | | SD | 4.713 | 0.590 | 1.571 | 4.680 | 0.089 |
| | | | | p-value | <0.0001*** | <0.0001*** | 0.001** | 0.983 | 0.001** |
| Healthy group | All specimens | | 43 | Mean | 27.29 | 3.40 | 0.26 | 8.57 | 0.03 |
| | | | | SD | 6.880 | 1.125 | 0.085 | 2.535 | 0.005 |

**[Table 4]**

| | | | A | B | C | D | E | I-1 | I-2 |
|---|---|---|---|---|---|---|---|---|---|
| | | | Trp | Kyn | 3-HAA | KA | AA | AA/KA | KA/AA |
| Hematological tumor group | All specimens | p-value | 1.04 × 10⁻⁵ | 3.71 × 10⁻³ | 5.92 × 10⁻⁴ | 3.00 × 10⁻⁸ | 2.57 × 10⁻⁰⁹ | 2.39 × 10⁻²² | 2.39 × 10⁻²² |

From Table 1, it has been found that the Trp amount and the KA amount in the serum are decreased in the hematological tumor group as compared with the healthy group. In addition, it has been found that the Kyn amount, the 3-HAA amount, and the AA amount are increased.

In particular, the fact that the KA amount is decreased in the hematological tumor group is not known in the related art including the non-patent document of C. Berthon et al., Leukemia Research 37 (2013) 573-579, and it is a finding that has been revealed for the first time in the present disclosure.

As shown in Tables 2 and 3, it has been found that the values of AA/Trp, AA/Kyn, AA/3-HAA, AA/KA, 3-HAA/Trp, 3-HAA/Kyn, and Kyn/Trp [Tables 2 and 3 (F) to (I-1), (M) to (O)] are increased in the hematological tumor group as compared with the healthy group.

On the other hand, it has been found that the values of KA/AA, KA/Trp, KA/Kyn, and KA/3-HAA [Tables 2 and 3 (I-2) and (J) to (L)] are decreased in the hematological tumor group as compared with the healthy group.

From Table 4, it can be seen that as compared with the healthy subjects, the decrease in the value of the Trp amount and the KA amount alone and the increase in the value of the AA amount alone in the hematological tumor group are more significant than the increase in the value of the Kyn amount and the 3-HAA amount alone.

That is, the P values indicating the significant difference in the decrease in the value of the Trp amount and the KA amount alone in the hematological tumor group were 1.04 × 10⁻⁵ and 3.00 × 10⁻⁸, respectively, and the P value indicating the significant difference in the increase in the value of the AA amount alone was 2.57 × 10⁻⁰⁹ [Table 4 (A, D, and E)]. On the other hand, the P values indicating the significance of the increase in the values of the Kyn amount and the 3-HAA amount in the hematological tumor group were 3.71 × 10⁻³ and 5.92 × 10⁻⁴, respectively [Table 4 (B) and (C)]. The P value indicating the significance of the increase of each of these amounts is a value of approximately 1/10 or more of the P value indicating the significant difference in the decrease in the value of the KA amount alone and the increase in the value of the AA amount alone.

As a result, it has been found that the KA amount and the AA amount are more useful as an indicator for distinguishing the hematological tumor group from the healthy group, and the KA amount is particularly useful.

Further, the P values of AA/KA, KA/AA, KA/AA, KA/Kyn, KA/3-HAA, and KA/Trp were all 0.001 or less [Table 2 and 3 (I-1) to (L)]. In particular, it has been found that the P values of AA/KA and KA/AA are 2.39 × 10⁻²², which is the lowest, and the P values of AA/KA and KA/AA are significantly decreased in all the classification types of the hematological tumor [Table 4 (I-1) and (I-2)].

As a result, it has been found that a specific combination of KA and a Trp-related substance, that is, a combination of AA/KA, KA/AA, KA/Kyn, KA/3-HAA, and KA/Trp is useful for distinguishing the hematological tumor group from the healthy group, and among them, AA/KA, KA/AA, KA/Kyn, and KA/3-HAA are useful, and AA/KA and KA/AA are the most useful.

In other words, it has been found it is possible to carry out a determination for a hematological tumor with a higher degree of certainty (accuracy and precision) by using the amounts of AA/KA, KA/AA, KA/Kyn, KA/3-HAA, Trp/KA, and KA as an indicator, and as the indicator, AA/KA, KA/AA, KA/Kyn, and KA/3-HAA are more preferable, and AA/KA and KA/AA are particularly preferable.

In addition, it is also possible to determine the effect of the medical treatment for the hematological tumor by monitoring changes in the KA amount and the values of AA/KA, KA/AA, KA/Kyn, KA/3-HAA, and Trp/KA, which are calculated by the determination method according to the embodiment of the present invention.

According to the method of assisting diagnosis of a hematological tumor of the present invention, it is possible to diagnose a hematological tumor with an excellent degree of certainty (sensitivity and specificity).

## Claims

1. A method of assisting diagnosis of a hematological tumor, comprising:
using, as an indicator, a decrease in an amount of kynurenic acid in a subject-derived specimen; or
using, as an indicator, a ratio between at least one amount selected from the group consisting of an amount of anthranilic acid, an amount of kynurenine, an amount of 3-hydroxyanthranilic acid, an amount of 3-hydroxykynurenine, and an amount of tryptophan, and the amount of kynurenic acid in the subject-derived specimen.

2. The method of assisting diagnosis of a hematological tumor according to claim 1,
wherein the ratio is used as an indicator.

3. The method of assisting diagnosis of a hematological tumor according to claim 1, comprising:
the following (1), (2), and (3);
(1) measuring A which is the amount of kynurenic acid, and B which is at least one amount selected from the group consisting of the amount of anthranilic acid, the amount of kynurenine, the amount of 3-hydroxyanthranilic acid, the amount of 3-hydroxykynurenine, and the amount of tryptophan in the subject-derived specimen,
(2) (i) calculating B/A, which is a ratio of B to A obtained in the (1), or (ii) calculating A/B, which is a ratio of A to B obtained in the (1), and
(3) providing an indicator for diagnosing whether or not a subject has a hematological tumor based on a result of the (2).

4. The method of assisting diagnosis of a hematological tumor according to claim 3, further comprising:
comparing at least one of the A/B or the B/A with a reference value.

5. The method of assisting diagnosis of a hematological tumor according to claim 3,
wherein the (2) is calculating a ratio of the amount of kynurenic acid to the amount of anthranilic acid, or a ratio of the amount of anthranilic acid to the amount of kynurenic acid.

6. The method of assisting diagnosis of a hematological tumor according to claim 1,
wherein the hematological tumor is a lymphatic hematological tumor or a myeloid hematological tumor.

7. The method of assisting diagnosis of a hematological tumor according to claim 1,
wherein the hematological tumor is a lymphatic hematological tumor.

8. The method of assisting diagnosis of a hematological tumor according to claim 6,
wherein the lymphatic hematological tumor is adult T-cell leukemia, diffuse large B-cell lymphoma, extranodal NK/T-cell lymphoma, nasal type follicular lymphoma, mucosal-associated lymphoid tissue lymphoma, mantle cell lymphoma, or Hodgkin's lymphoma.

9. The method of assisting diagnosis of a hematological tumor according to claim 1,
wherein the specimen is serum, blood plasma, or whole blood.

10. A method of obtaining data for diagnosis of a hematological tumor, the method comprising:
the following (1) and (2);
(1) measuring A which is an amount of kynurenic acid or measuring A which is an amount of kynurenic acid and B which is at least one amount selected from the group consisting of an amount of anthranilic acid, an amount of kynurenine, an amount of 3-hydroxyanthranilic acid, an amount of 3-hydroxykynurenine, and an amount of tryptophan in a subject-derived specimen, and
(2) (i) using A obtained in the (1) as data for diagnosing a hematological tumor, (ii) calculating B/A, which is a ratio of B to A obtained in the (1), and using the calculated B/A as data for diagnosing a hematological tumor, or (iii) calculating A/B, which is a ratio of A to B obtained in the (1), and using the calculated A/B as data for diagnosing a hematological tumor.

11. A kit for carrying out the method of assisting diagnosis of a hematological tumor according to any one of claims 1 to 9 or the method of obtaining data for diagnosis of a hematological tumor according to claim 10, the kit comprising:
a mobile phase; and
a column.

12. A kit for carrying out the method of assisting diagnosis of a hematological tumor according to any one of claims 1 to 9 or the method of obtaining data for diagnosis of a hematological tumor according to claim 10, the kit comprising:
a substance having an affinity for kynurenic acid; or
a substance having an affinity for kynurenic acid and
at least one substance selected from the group consisting of a substance having an affinity for anthranilic acid, a substance having an affinity for kynurenine, a substance having an affinity for 3-hydroxyanthranilic acid, a substance having an affinity for 3-hydroxykynurenine, and a substance having an affinity for tryptophan.
